# NEW EUROPEAN PATENT SPECIFICATION

(11) **EP 2 249 842 B2**
(45) Date of publication and mention of the opposition decision: **05.03.2025**
(45) Mention of the grant of the patent: 27.07.2016
(21) Application number: 09710537.3
(22) Date of filing: 12.02.2009
(51) Int. Cl.: A61K 31/593, A61K 31/59, A23L 33/155, A61P 21/00, A61P 43/00, A61P 21/06

(54) **USE OF 25-HYDROXY-VITAMIN D3 AND VITAMIN D TO AFFECT HUMAN MUSCLE PHYSIOLOGY**
VERWENDUNG VON 25-HYDROXY-VITAMIN D3 UND VITAMIN D ZUR EINWIRKUNG AUF DIE MENSCHLICHE MUSKELPHYSIOLOGIE
UTILISATION DE 25-HYDROXY-VITAMINE D3 ET VITAMINE D POUR AGIR SUR LA PHYSIOLOGIE MUSCULAIRE

(30) Priority: 13.02.2008 US 28510 P; 26.02.2008 US 31671 P; 14.03.2008 US 36924 P; 15.03.2008 US 36928 P; 06.06.2008 US 129139 P
(43) Date of publication of application: 17.11.2010
(73) Proprietor: DSM IP Assets B.V., 6411 TE Heerlen (NL)
(72) Inventor: BUCK, Neil, Robert, F-68220 Leymen (FR); CLAERHOUT, Wouter, CH-4102 Binningen (CH); LEUENBERGER, Bruno, H., CH-4123 Rheinfelden (CH); STOECKLIN, Elisabeth, CH-4144 Arlesheim (CH); URBAN, Kai, 79713 Bad Säckingen (DE); WOLFRAM, Swen, 79761 Waldshut-Tiengen (DE)
(74) Representative: Kraus & Lederer PartGmbB
(86) International application number: PCT/EP2009/051641
(87) International publication number: WO 2009/101137

(56) References cited:
- WO-A1-2007/059960
- WO-A2-2007/092755
- WO-A2-2009/047644
- US-A1- 2003 170 324
- US-A1- 2007 082 089
- MONTERO-ODASSO M ET AL: "Vitamin D in the aging musculoskeletal system: An authentic strength preserving hormone", MOLECULAR ASPECTS OF MEDICINE, PERGAMON PRESS, OXFORD, GB, vol. 26, no. 3, 1 June 2005 (2005-06-01), pages 203 - 219, XP025272601, ISSN: 0098-2997, [retrieved on 20050601], DOI: 10.1016/J.MAM.2005.01.005
- VISSER MARJOLEIN ET AL: "Low vitamin D and high parathyroid hormone levels as determinants of loss of muscle strength and muscle mass (sarcopenia): The Longitudinal Aging Study Amsterdam", JOURNAL OF CLINICAL ENDOCRINOLOGY AND METABOLISM, THE ENDOCRINE SOCIETY, US, vol. 88, no. 12, 1 December 2003 (2003-12-01), pages 5766 - 5772, XP002502110, ISSN: 0021-972X, DOI: 10.1210/JC.2003-030604
- STAMP T C B ET AL: "Comparison of oral 25-hydroxycholecalciferol, vitamin D, and ultraviolet light as determinants of circulating 25-hydroxyvitamin D", THE LANCET, LANCET LIMITED. LONDON, GB, vol. 1, no. 8026, 25 June 1977 (1977-06-25), pages 1341 - 1343, XP002526420, ISSN: 0140-6736, DOI: 10.1016/S0140-6736(77)92553-3
- ZITTERMANN ET AL: "Vitamin D and disease prevention with special reference to cardiovascular disease", PROGRESS IN BIOPHYSICS AND MOLECULAR BIOLOGY, PERGAMON PRESS, OXFORD, GB, vol. 92, no. 1, 1 September 2006 (2006-09-01), pages 39 - 48, XP025078429, ISSN: 0079-6107, [retrieved on 20060901], DOI: 10.1016/J.PBIOMOLBIO.2006.02.001
- WICHERTS ET AL.: "Vitamin D Status Predicts Physical Performance and Its Decline in Older Persons", J CLIN ENDOCRINOL METAB, vol. 92, 2007, pages 2058 - 2065
- JETTER ET AL.: "Pharmacokinetics of oral vitamin D3 and calcifediol", BONE, vol. 59, 2014, pages 14 - 19
- RIMANIOL ET AL.: "Muscle weakness in intensive care patients: Initial manifestation of vitamin D deficiency", INTENSIVE CARE MED, vol. 20, 1994, pages 591 - 592
- BATEMAN, T.A. ET AL: "Osteoprotegerin mitigates tail suspension-induced osteopenia", BONE, PERGAMON PRESS., OXFORD, GB, vol. 26, no. 5, 1 May 2000 (2000-05-01), GB , pages 443 - 449, ISSN: 8756-3282, DOI: 10.1016/S8756-3282(00)00256-8
- GAO YUNFANG ET AL: "Muscle Atrophy Induced by Mechanical Unloading: Mechanisms and Potential Countermeasures", FRONTIERS IN PHYSIOLOGY, FRONTIERS RESEARCH FOUNDATION, CH, vol. 9, CH , pages 1 - 9, ISSN: 1664-042X, DOI: 10.3389/fphys.2018.00235
- WHYTE M P ET AL: "Rickets and osteomalacia", MEDICINE,, vol. 33, no. 12, 1 December 2005 (2005-12-01), pages 70 - 74, XP025344242, ISSN: 1357-3039, [retrieved on 20051201]
- DATABASE BIOSIS [online] BIOSCIENCES INFORMATION SERVICE, PHILADELPHIA, PA, US; 1997, GARAY LILLO J ET AL: "Geminis Project: Prospective, multicentric and random study for evaluating the effect of tricalcium phosphate versus tricalcium phosphate plus 25 (OH) vitamin D on the risk of fractures in elderly women", XP002525526, Database accession no. PREV199799742655
- GERIATRIKA (MADRID), vol. 13, no. 6, 1997, pages 24 - 28, ISSN: 0212-9744
- EASTWOOD J B ET AL: "THE EFFECT OF 25 HYDROXY VITAMIN D3 IN THE OSTEOMALACIA OF CHRONIC RENAL FAILURE", CLINICAL SCIENCE AND MOLECULAR MEDICINE, BLACKWELL SCIENTIFIC PUBLICATIONS, OXFORD, GB, vol. 52, no. 5, 1 January 1977 (1977-01-01), pages 499 - 508, XP009085521, ISSN: 0301-0538
- DATABASE MEDLINE [online] US NATIONAL LIBRARY OF MEDICINE (NLM), BETHESDA, MD, US; 1981, BARBIZET J ET AL: "[Myopathy, osteomalacia, and congenital hypoalbuminemia (author's transl)]", XP002526397, Database accession no. NLM7256069
- REVUE NEUROLOGIQUE 1981, vol. 137, no. 3, 1981, pages 195 - 202, ISSN: 0035-3787
- DATABASE MEDLINE [online] US NATIONAL LIBRARY OF MEDICINE (NLM), BETHESDA, MD, US; November 1994 (1994-11-01), RIMANIOL J M ET AL: "Muscle weakness in intensive care patients: initial manifestation of vitamin D deficiency.", XP002526398, Database accession no. NLM7706575
- INTENSIVE CARE MEDICINE NOV 1994, vol. 20, no. 8, November 1994 (1994-11-01), pages 591 - 592, ISSN: 0342-4642
- GRANT ET AL: "Epidemiology of disease risks in relation to vitamin D insufficiency", PROGRESS IN BIOPHYSICS AND MOLECULAR BIOLOGY, PERGAMON PRESS, OXFORD, GB, vol. 92, no. 1, 1 September 2006 (2006-09-01), pages 65 - 79, XP025078432, ISSN: 0079-6107, [retrieved on 20060901]
- SATO YOSHIHIRO ET AL: "Low-dose vitamin D prevents muscular atrophy and reduces falls and hip fractures in women after stroke: a randomized controlled trial.", CEREBROVASCULAR DISEASES (BASEL, SWITZERLAND) 2005, vol. 20, no. 3, 2005, pages 187 - 192, XP009115855, ISSN: 1015-9770
- BISCHOFF-FERRARI ET AL: "Vitamin D and muscle function", INTERNATIONAL CONGRESS SERIES, EXCERPTA MEDICA, AMSTERDAM, vol. 1297, 1 March 2007 (2007-03-01), pages 143 - 147, XP022003749, ISSN: 0531-5131
- PFEIFER M ET AL: "Vitamin D and muscle function.", OSTEOPOROSIS INTERNATIONAL : A JOURNAL ESTABLISHED AS RESULT OF COOPERATION BETWEEN THE EUROPEAN FOUNDATION FOR OSTEOPOROSIS AND THE NATIONAL OSTEOPOROSIS FOUNDATION OF THE USA MAR 2002, vol. 13, no. 3, March 2002 (2002-03-01), pages 187 - 194, XP002526396, ISSN: 0937-941X
- BARGER-LUX M J ET AL: "Vitamin D and its major metabolites: Serum levels after graded oral dosing in healthy men", OSTEOPOROSIS INTERNATIONAL, XX, XX, vol. 8, no. 3, 1 January 1998 (1998-01-01), pages 222 - 230, XP002438418

## Description

### FIELD OF THE INVENTION

### BACKGROUND OF THE INVENTION

The present invention relates to use of 25-hydroxyvitamin D3 (calcifediol, 25-OH D3) and Vitamin D3 in the manufacture of a pharmaceutical, nutraceutical, food supplement or food composition to retain or prevent the loss of muscle strength, muscle function, or both in an elderly person or a person who suffers chronic immobility. Vitamin D3 (cholecalciferol) is used together with 25-OH D3.

Vitamin D (e.g., ergocalciferol and cholecalciferol) is a group of fat-soluble compounds defined by their biological activity. A deficiency of vitamin D causes rickets in children and osteomalacia in adults. But toxicity can occur after chronic intake of more than 100 times the recommended daily allowance (i.e., 5-15 µg or 200-600 IU vitamin D) for several months. For vitamin D, "The threshold for toxicity is 500 to 600 mcg/kg body weight per day. In general, adults should not consume more than three times the RDA for extended period of time" (Garrison & Somer, The Nutrition Desk Reference, Third Ed., McGraw-Hill, pg. 82, 1997). Hypercalcemia may occur at a blood concentration of 25-hydroxyvitamin D greater than 375 nmol/L. More recently, a safe upper level of Vitamin D was identified to be at least 250 µg/ day (10'000 IU) (Hathcock et aL Am.J Clin. Nutr. 85:6-18, 2007). Ingestion of such as a dietary supplement has been shown to result in a blood concentration of about 200 nmol/L 25-hydroxyvitamin D.

Vitamin D is a prohormone which has to be hydroxylated in the liver to produce 25-hydroxyvitamin D (calcifediol; 25-OH vitamin D; 25-OH D), which then undergoes another hydroxylation in the kidney and other tissues to produce 1,25-dihydroxyvitamin D, the active hormone form of vitamin D. 1,25-dihydroxyvitamin D is released into the blood, binds to vitamin D binding protein (DBP), and is transported to target tissues. Binding between 1,25-dihydroxyvitamin D and vitamin D receptor allows the complex to act as a transcription factor in the cell's nucleus.

Vitamin D deficiency may promote resorption of bone. It may also modulate function of the cardiovascular, immune, and muscular systems. Epidemiological studies find associations between vitamin D intake and its effect on blood pressure or glucose metabolism. The activity of vitamin D is under negative feedback control by parathyroid hormone.

Both Vitamin D and 25-OH D3 have been administered as pharmaceuticals in the past. Vitamin D, is of course widely available; 25-OH D3 was previously sold in the USA by Organon USA under the name "CALDEROL", but is currently on the FDA's list of discontinued drugs. It was a gelatine capsule containing corn oil and 25-OH D3.

A liquid form of 25-OH D3 is currently sold in Spain by FAES Farma under the name "HIDROFEROL" in an oil solution.

The combination of vitamin D and 25-OH D3 has been used in animal feed. 25-OH D3 for use in feed is commercially available from DSM under the name "ROVIMIX HY-D".

Tritsch et al. (US 2003/0170324) disclose a feed premix composition of at least 25-OH D3 in an amount between 5% and 50% (wt/wt) dissolved in oil and an antioxidant, an agent encapsulating droplets of 25-OH D3 and oil, and a nutritional additive (e.g., Vitamin D3). The premix may be added to poultry, swine, canine, or feline food. This composition stabilizes 25-OH D3 against oxidation.

Simoes-Nunes et al. (US 2005/0064018) discloses adding a combination of 25-OH Vitamin D3 and Vitamin D3 to animal feed. In particular, about 10 µg/kg to about 100µg/kg of 25-OH Vitamin D3 and about 200 IU/kg to about 4,000 IU/kg of Vitamin D3 are added to swine feed. This addition improves the pig's bone strength.

Stark et al. (US 5,695,794) disclose adding a combination of 25-OH Vitamin D3 and Vitamin D3 to poultry feed to ameliorate the effects of tibial dyschondroplasia.

Borenstein et al US 5,043,170 discloses the combination of Vitamin D3 and either 1-alpha-hydroxycholecalciferol or 1 alpha, 25-dihydroxycholecalciferol to improve egg strength and leg strength in laying hens and older hens.

Chung et al, WO 2007/059960 discloses that sows fed a diet containing both Vitamin D3 and 25-hydroxVitamin D3 had improved general health status, body frame, litter size and health, and other production parameters. Also a 25-OH D3 human food supplement is disclosed, but its dosage range, 5-15 micrograms per kg body weight, which equals to an extremely high daily dosage of 300-900 micrograms per human is very high.

Case 26706-EP-EPT PCT/EP08/006357 discloses that prenatal exposure of piglets to 25-OH D3 (by feeding the pregnant sow) enhances muscle development in the offspring.

Sato et al 2005 Cerebrovasc Dis 20:187-192 discusses Vitamin D therapy for prevention of falls in stroke survivors who were Vitamin D deficient. There is no mention of combining Vitamin D with 25-OH D3.

Bischoff-Ferrari 2007 Int. Congress Ser. 1297:143-147 recommends the use of vitamin d to prevent falls and ultimately fractures in the institutionalized elderly. There is no suggestion of combining this with 25-OH D3.

To our knowledge the prior art does not teach or suggest use of the combination of 25-OH D3 and vitamin D as a medicament for humans to increase muscle strength, muscle function, or both. Other advantages and improvements are described below or would be apparent from the disclosure herein.

### Summary of the Invention

The invention provides the use of 25 hydroxyvitamin D3 ("25-OH D3") and Vitamin D3 in the manufacture of a pharmaceutical, nutraceutical, food supplement or food composition to retain or prevent the loss of muscle function or muscle strength in a human, where the human is an elderly person or a person who suffers chronic immobility regardless of age.

It has been found, in accordance with this invention, that daily or weekly treatment with 25-OH D3 surprisingly results in improvements of muscle strength and/or function compared to consumption of identical dosages of Vitamin D.

According to the invention, 25-OH-D3 is useful as a medicament to increase muscle strength and/or function in a human. The medicament further comprises vitamin D3. Forms and dosages of a pharmaceutical composition, as well as processes for manufacturing medicaments, are also disclosed.

Vitamin D3 may be administered together with or separately from 25-OH D3. They may be administered once per day, once per week, or once per month. Generally, the administration period is at least for one month, preferably for more than two months, and more preferably for at least four months so that changes in muscle strength can be clearly observed. Strength may be measured using art-recognized tests, such as knee flexor and extensor strength tests.

In another aspect of the disclosure, a method of increasing muscle function by administering an effective amount of 25-OH D3 and vitamin D3 is provided. Optionally, vitamin D may be administered together with or separately from 25-OH D3. They may be administered once per day, once per week, or once per month. Muscle function may be assessed by art-recognized tests, such as the repeated sit-to-stand test, and the timed up and go test.

In another aspect of the disclosure, a pharmaceutical composition suitable for human use is provided which comprises vitamin D3, 25-OH D3, and a pharmaceutically acceptable carrier in muscle strengthening amounts.

Further, it has been found, in accordance with this invention that the combination of 25-OH D3 and Vitamin D3 synergistically regulates (either up-regulates or down-regulates) a synergistic number of Vitamin D responsive skeletal muscle genes, including a high number of genes which are not responsive to the presence of either Vitamin D3 nor 25-OH D3 alone. This is a surprising result, as it is not explained by the current model of Vitamin D metabolism, which postulates that virtually all Vitamin D is first metabolized into 25-OH D.

The combination, in accordance with this invention, provides two significant advantages:
1) It results in a rapid and synergistic plasma response of 25-OH D
2) It leads to an unexpectedly pronounced and long plateau of plasma 25-OH D levels. These are especially important goals of treatment of muscular disorders which are the consequence of a Vitamin D deficiency: a rapid correction of suboptimal Vitamin D status and a long and stable plasma concentration to ensure sufficient supply of 25-OH to muscle tissue.

### BRIEF DESCRIPTION OF THE FIGURES

Another aspect of this disclosure is a food, functional food, food supplement or nutraceutical for use in maintaining muscle strength and function for human consumption containing a combination of Vitamin D and 25-OH D3.

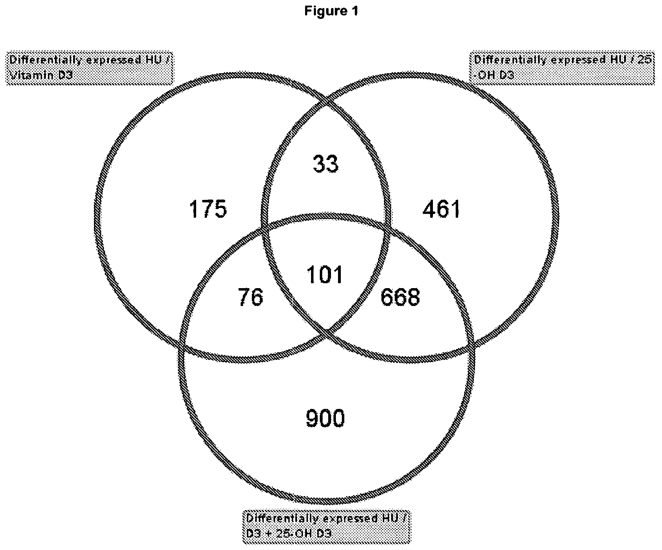

FIGURE 1 shows Venn Diagrams of differentially expressed probe sets for murine genes for the hindlimb unloaded ("HU group") and the treatment groups (Vitamin D3, 25-OH D3 or the combination).
FIGURE 2 shows Venn Diagrams of differentially expressed probe sets for genes between 25-OH D3 treatment group and the treatment group with the combination of 25-OH D3 + Vitamin D3.
FIGURE 3 shows Venn Diagrams of differentially expressed probe sets for genes between Vitamin D3 treatment group and the treatment group with the combination of 25-OH D3 + Vitamin D3.
FIGURE 4 is an enrichment analysis (performed with GeneGo MetaCore) of the 1745 probe sets for genes differentially expressed between the HU group and the group receiving a combination treatment of 25-OH D3 and Vitamin D3.

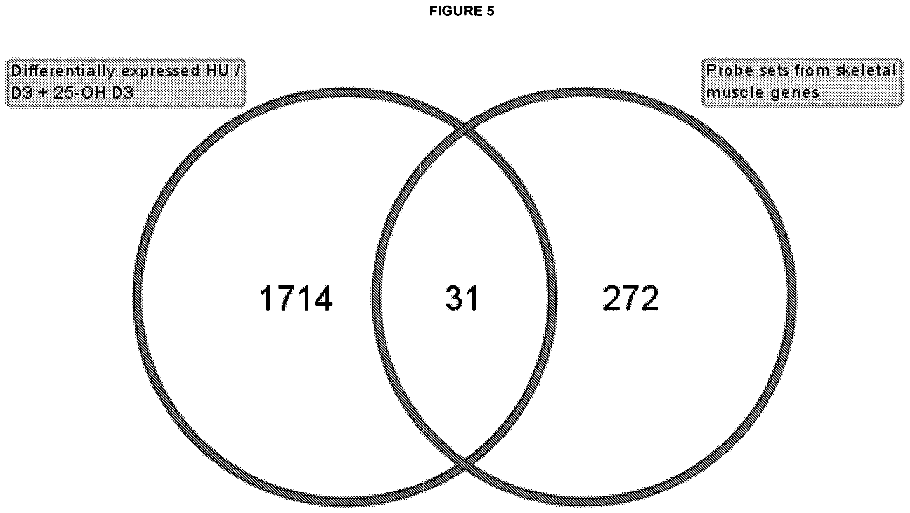

FIGURE 5 shows Venn Diagrams of differentially expressed probe sets in the 25-OH D3 + Vitamin D3 treatment group and probe sets for selected skeletal muscle genes.
FIGURE 6 shows Venn Diagrams of differentially expressed probe sets in the 25-Hydroxyvitamin D3 treatment group and probe sets for selected skeletal muscle genes.
FIGURE 7 shows Venn Diagrams of the differentially expressed probe sets in the Vitamin D3 treatment group and probe sets for selected skeletal muscle genes.

As used throughout the specification and claims, the following definitions apply:
"Vitamin D" means either Vitamin D3 (cholecalciferol) and/or Vitamin D2 (ergocaciferol). Humans are unable to make Vitamin D2 (ergocalciferol), but are able to use it as a source of Vitamin D. Vitamin D2 can be synthesized by various plants and is often used in Vitamin D in supplements as an equivalent to Vitamin D.

"Vitamin D metabolite" means any metabolite of Vitamin D other than 25-hydroxy vitamin D3.

"25-OH D3" refers specifically to 25-hydroxyvitamin D3

"25-OH D" refers to the 25-hydroxylated metabolite of either Vitamin D2 or Vitamin D3 which is the major circulating form found in plasma.

"Prevent" is meant to include amelioration of the disease, lessening of the severity of the symptoms, early intervention, and lengthening the duration of onset of the disease, and not intended to be limited to a situation where the patient is no longer able to contract the disease nor experience any symptoms.

Further disclosed herein is a kit which is comprised of multiple, separate dosages of Vitamin D or Vitamin D3 along with a dosage of 25-OH D3. They may be enclosed in a container: e.g., bottle, blister pack, or vial rack. Further, instructions for administering the composition as a dosage to a human are provided within the kit.

In another embodiment, the 25-OH D3 in combination with Vitamin D3 is the active ingredient to preserve healthy muscle strength or function in a food, functional food, food supplement or nutraceutical suitable for human consumption. The dosages of the 25-OH and D3 may be the same as those present in the pharmaceutical product, but preferably will tend towards the lower ranges. The food supplements and nutraceuticals may be in the form of tablets, capsules or other convenient dosage forms. The food may be a beverage or food, and if desired, may also contain other nutritionally effective compounds such as other vitamins, minerals, and the like.

Vitamin D deficiency is an especially prevalent condition in the elderly population and those who suffer chronic immobility regardless of age. This may be due to the general lack of exposure to sunlight, a lessened ability of the body to manufacture vitamin D or metabolize it efficiently, or a number of other causes. One consequence of Vitamin D deficiency is a loss of muscle strength and/or function. Thus one aspect of this invention is the use of the combination of Vitamin D3 and 25-OH D3 in an elderly population to maintain or prevent the loss of muscle strength and function. As used throughout, the term "elderly" is meant to encompass those individuals who are over 65 years of age, preferably over 70, and even over 80.

In the present disclosure, this combination of 25-OH D3 and Vitamin D3 is suitable to maintain or prevent the loss of muscle strength and function in people who are at risk of developing muscle strength and or function conditions characterized by Vitamin D deficiency or insufficiency. This would include especially adults, including postmenopausal women (i.e. about age 45 and older) and men who are about age 45 and older. It is especially suitable for individuals who do not receive a great deal of natural sunlight exposure, such as for people who traditionally wear long clothing, do not go out of doors regularly, or who use sunscreens when they are exposed to sunlight, or live in geographical areas significantly north or south of the equator, where sunlight is less intense.

The present disclosure is useful in a method to maintain, prevent the loss of, and/or restore healthy muscle strength and function in a human with a malabsorption syndrome (e.g., affected by celiac disease, sprue, or short bowel syndrome), and it thereby at risk of Vitamin D deficiency by administering the combination of Vitamin D and 25-OH D3.

The present disclosure is useful in a method to maintain, prevent the loss of, and/or restore healthy muscle strength and function in a human with impaired liver function, wherein the human cannot process Vitamin D into 25-hydroxyvitamin D efficiently, by providing the human with a combination of Vitamin D and 25-hydroxyvitamin D3.

The compositions for use according to the invention are also beneficial for the retention of muscle mass in the elderly (up to 80 years old) and the very elderly (80 or above years old), particularly those who are in institutionalized care facilities (e.g., hospital, nursing home, rehabilitation clinic, elder assisted living), or those with muscle atrophy.

In another embodiment 25-OH D3 and Vitamin D3 for use according to the invention maintain or prevent loss of muscle strength in the elderly. Loss of muscle strength is also a major cause of falls in the elderly and may contribute to the high number of falls that take place in the hospital. For an older person who has diminished physiologic reserves but still can perform daily activities (such as walking, bathing and toileting functions), the accelerated losses of muscle strength after even a few days bed rest may result in a prolonged loss if independent function. Even if this loss is eventually reversed, rehabilitation requires extensive and expensive intervention because reconditioning of muscles takes considerably longer than the deconditioning process.

The compositions for use according to the invention are also beneficial for the retention and/or increase of muscle mass in people who may not be elderly, but who lose muscle mass because they are immobilized due to another condition. As explained by the Merck Manual of Geriatrics, 2nd Ed: (p316-318): with complete inactivity, muscle strength decreases by 5% per day. Even young men on bed rest lose muscle strength at a rate of 1.0% to 1.5% per day (or about 10% per week).

Thus the compositions for use according to this invention are beneficial for people who are subject to loss of muscle mass due to decreased mobility, or who are even immobile. The cause of the decreased mobility does not matter in the practice of this invention, as the goal here is to protect against loss of muscle mass. For example, the loss of mobility may be from trauma, stroke, being in a cast, Parkinson's disease, multiple sclerosis, myasthenia gravis, or even Creutzfeldt-Jacobs disease. Thus another aspect is to administer the compounds for use according to of this invention to a paraplegic, or a quadriplegic individual.

The composition for use described herein are also beneficial for those suffering from cachexia or sarcopenia. Cachexia is a "body-wasting" syndrome that is a co-morbidity with cancers and AIDS. Other syndromes or conditions which can induce skeletal muscle atrophy are congestive heart disease, chronic obstructive pulmonary disease, liver disease, starvation, burns, etc. Sarcopenia is another condition (distinct from cachexia and atrophy) which relates to an age-related decrease in muscle function. The exact cause is unknown.

This present disclosure also relates to benefiting those with muscle weakness. Muscle weakness is the physical part of fatigue (medical). Locations for muscle weakness are central, neural and peripheral. Central muscle weakness is an overall exhaustion of the whole body, while peripheral weakness is an exhaustion of individual muscles. Neural weakness is somewhere between.

Muscle weakness may be due to problems with the nerve supply or problems with muscle itself. The latter category includes polymyositis and other muscle disorders e.g. amyotrophic lateral sclerosis, botulism, centronuclear myopathy, myotubular myopathy, dysautonomia, Charcot-Marie-Tooth, hypokalemia, motor neurone disease, muscular dystrophy, myotonic dystrophy, myasthenia gravis, progressive muscular atrophy, spinal muscular atrophy, cerebral palsy, infectious mononucleosis, herpes zoster, vitamin D deficiency, fibromyalgia, celiac disease, hypercortisolism (Cushing's syndrome), hypocortisolism (Addison's disease), primary hyperaldosteronism (Conn's syndrome), and diarrhea.

### Dosage Forms

Vitamin D3 and 25-OH D3 may be obtained from any source, and a composition thereof may be prepared using convenient technology. In general, crystals of vitamin D3, 25-OH D3, or both (separately or together) are dissolved in an oil with heating and agitation. Preferably, the oil is transferred into a vessel and heated. Thereafter, vitamin D3, 25-OH D3, or both are added to the vessel, while maintaining the temperature of the oil or increasing it over time. The composition is agitated to dissolve the crystals of vitamin D3, 25-OH D3, or both. Prior to addition to the oil, the crystals may be reduced in size by milling and/or sieving, to enhance dissolving. The composition may be agitated by stirring, vessel rotation, mixing, homogenization, recirculation, or ultrasonication. Preferably, the oil may be heated in the vessel to a temperature from about 80°C to about 85°C, sized crystals are introduced into the vessel, and the contents are stirred to dissolve the crystals into the oil.

The "oil" may be any edible oil, lipid, or fat: e.g., babassu oil, coconut oil, cohune oil, murumyru tallow, palm kernel oil, or tucum oil. The oil may be natural, synthetic, semisynthetic, or any combination thereof. Natural oil may be derived from any source (e.g., animal, plant, fungal, marine); synthetic or semisynthetic oil may be produced by convenient technology. Preferably, the oil is a mixture of plant medium chain triglycerides, mainly caprylic and capric acids. The composition may optionally contain one or more other suitable ingredients such as, for example, and a pharmaceutically acceptable antioxidant, preservatives, dissolution agents, surfactants, pH adjusting agents or buffers, humectants, and any combination thereof. The foregoing are examples of pharmaceutically acceptable carriers.

Suitable antioxidants include tocopherol, mixed tocopherols, tocopherols from natural or synthetic sources, butylated hydroxy toluene (BHT), butylated hydroxy anisole (BHA), natural antioxidants like rosemary extract, propyl galate, and any others used in the manufacture of pharmaceuticals for humans. Preferably, the antioxidant is tocopherol. Suitable preservatives include methyl paraben, propyl paraben, potassium sorbate, sodium benzoate, benzoic acid, and any combination thereof. Suitable dissolution agents include inorganic or organic solvents: e.g., alcohols, chlorinated hydrocarbons, and any combination thereof. Suitable surfactants may be anionic, cationic, or nonionic: e.g., ascorbyl palmitate, polysorbates, polyethylene glycols, and any combination thereof. Suitable pH adjusting agents or buffers include citric acid-sodium citrate, phosphoric acid-sodium phosphate, acetic acid-sodium acetate, and any combination thereof. Suitable humectants include glycerol, sorbitol, polyethylene glycol, propylene glycol, and any combination thereof.

Once formed, the oil composition may be incorporated in various other useful compositions, some of which are discussed below. For example, emulsions may be formed, which may be optionally encapsulated or spray dried. A variety of emulsions may be prepared by combining the nonaqueous compositions described above with an aqueous composition. The emulsion may be of any type. Suitable emulsions include oil-in-water emulsions, water-in-oil emulsions, anhydrous emulsions, solid emulsions, and microemulsions. The emulsions may be prepared by any convenient technology. The emulsion contains an aqueous composition and a nonaqueous (e.g., oil) composition, wherein the latter comprises vitamin D3, 25-OH D3, or both (separately or together) dissolved in an oil in an amount of between about 3% and about 50% by weight based on the total weight of the oil composition. As used herein, "aqueous composition" and "aqueous phase" are used interchangeably. Generally, the emulsion may contain from about 20% to about 95% of an aqueous composition, and from about 5% to about 80% of a nonaqueous composition. Preferably, however, the emulsion contains from about 85% to about 95% (vol/vol) of an aqueous composition, and from about 5% to about 15% (vol/vol) of a nonaqueous composition. Conveniently, the nonaqueous composition may be dispersed as droplets in the aqueous composition. For example, the droplets may have a mean diameter of less than about 500 nm in the aqueous composition. Conveniently, the droplets have a mean diameter of between about 150 nm and about 300 nm.

In a particularly advantageous embodiment, the emulsion contains an encapsulating agent, which facilitates encapsulating the oil composition upon further processing of the emulsion (e.g., by spray drying). The encapsulating agent may be any edible substance capable of encapsulating the oil composition. Preferably, the encapsulation agent is predominantly a colloidal material. Such materials include starches, proteins from animal sources (including gelatins), proteins from plant sources, casein, pectin, alginate, agar, maltodextrins, lignin sulfonates, cellulose derivatives, sugars, saccharides, sorbitols, gums, and any combination thereof.

Suitable starches include: plant starches (e.g., CAPSUL^{®} or HI-CAP^{®} from National Starch & Chemical Corp., New York, NY), other modified food starches, and any combination thereof. Preferably, the starch is CAPSUL^{®} modified plant starch. Suitable proteins from animal sources include: gelatins (e.g., bovine gelatins, porcine gelatins (Type A or B) with different Bloom numbers, fish gelatins), skim milk protein, caseinate, and any combination thereof. Preferably, the animal protein is a gelatin. Suitable proteins from plant sources include: potato protein (e.g., ALBUREX^{®} from Roquette Preres Societe Anonyme, Lestrem, France), pea protein, soy protein, and any combination thereof. Preferably, the plant protein is ALBUREX^{®} potato protein. Suitable maltodextrins with a different dextrose equivalent include: maltodextrin 5, maltodextrin 10, maltodextrin 15, maltodextrin 20, maltodextrin 25, and any combination thereof. Preferably, the maltodextrin is maltodextrin 15. Suitable cellulose derivatives include: ethyl cellulose, methylethyl cellulose, hydroxypropyl cellulose, hydroxypropylmethyl cellulose, carboxymethylcellulose, and any combination thereof. Suitable saccharides include lactose, sucrose, or any combination thereof. Preferably, the saccharide is sucrose. Suitable gums include: acacia, locust bean, carragean, and any combination thereof. Preferably, the gum is gum acacia.

When the emulsion contains an encapsulating agent, the encapsulating agent may be dispersed in water by any convenient technology to form an aqueous phase. The aqueous phase may be a solution or a mixture depending on the properties of the components selected. The selected components may be dispersed by any convenient technology including: homogenizing, mixing, emulsifying, recirculating, static mixing, ultrasonication, stirring, heating, or any combination thereof. The viscosity of the resulting aqueous phase may then be adjusted, as desired, by the addition of water. The aqueous composition of the emulsion may optionally contain any other suitable material including but not limited to, those discussed above in reference to the nonaqueous composition. Preferably, the aqueous composition may include, an encapsulating agent, a film-forming agent, a plasticizer, a preservative, an antioxidant, or any combination thereof. Suitable preservatives include methyl paraben, propyl paraben, sorbic acid, potassium sorbate, sodium benzoate, and any combination thereof. Suitable antioxidants include sodium ascorbate, ascorbic acid, citric acid, and any combination thereof.

Preferably, the aqueous phase contains a modified food starch, such as octenyl succinyl starch (CAPSUL^{®}), maltodextrin, and sodium ascorbate. Another preferred aqueous phase contains potato protein (ALBUREX^{®}), maltodextrin 20, and sodium ascorbate. The selected components may be dissolved in water by any convenient technology, preferably stirring. The mixture is preferably homogenized until it is uniform and lump free. Preferably, the homogenization is carried out at a temperature between about 50°C and about 75°C. The final viscosity of the resulting aqueous phase may then be adjusted to the desired viscosity, preferably about 250 cp to about 450 cp, more preferably about 300 cp to about 400 cp, even more preferably about 385 cp.

The emulsion may be formed by emulsifying the nonaqueous composition and the aqueous phase by any means, including homogenization, rotor-stator shear, high pressure shear and cavitation, high speed "cowles" or shear agitation, and any combination thereof. The volume and viscosity of the emulsion may preferably be adjusted by the addition of water after emulsification. Preferably, the nonaqueous and aqueous compositions are emulsified by homogenization. Preferably, the emulsion should not contain any mineral, transition metal, or peroxide.

As noted above, the emulsion may be incorporated or employed in producing other useful compositions, especially encapsulated oils, e.g., spray-dried powders. Generally, the encapsulated oil comprises an oil composition and an encapsulation agent encapsulating the oil composition, wherein the oil composition contains vitamin D3, 25-OH D3, or both dissolved in the oil in an amount between about 3% and about 50% by weight based on the total weight of the oil composition. The encapsulated oil may be produced by any convenient technology: e.g., drying an emulsion described above by any conventional technology, including spray drying, freeze drying, fluid bed drying, tray drying, adsorbtion, and any combination thereof. Preferably, the encapsulated oil is produced by spray drying an emulsion having an aqueous phase above containing an encapsulation agent; spray drying parameters are dictated by the physical characteristics desired in the final encapsulated oil. Such physical parameters include particle size, powder shape and flow, and water content. Preferably, the oil is in an amount less than about 30%, less than about 20%, less than about 10%, or less than about 5% by weight based on the total weight of the encapsulated oil. The encapsulated oil should have good flowability and the vitamin D3 and/or 25-OH D3 should be distributed homogeneously throughout the composition. Conveniently, the encapsulated oil is a powder. Any other suitable additive may be added to the encapsulated oil. One such additive may be a flow agent such as silicon dioxide, to increase the flowability of the encapsulated oil.

The composition may be provided in the form of a tablet, capsule (e.g., hard or soft), or injection (e.g., oil or emulsion). They may be packaged in a single daily dosage.

### Dosages

*Daily.* A composition according to this disclosure where the two active ingredients are to be administered separately, contains Vitamin D or 25-OH D3 in an amount from about 1 µg to about 50 µg, preferably about 5 µg and 25 µg. Alternatively, a single daily dosage having both Vitamin D and 25-OH D3 contains each active ingredient in an amount from about 1 µg to about 50 µg, preferably about 5 µg and 25 µg.

The dosage ratio of Vitamin D to 25-OH D3 may be from about 50:1 to about 1:50, more preferably from about 25:1 to about 1:25, and even more preferably from about 6:1 to about 1:6.

Multiple, separate dosages may be packaged in a single kit (or container). For example, the kit may be comprised of thirty separate daily dosages of both actives separately (i.e. 60 separate dosages), or combined (i.e. 30 dosages containing both active ingredients). Instructions for administering the dosages to a human may be included in the kit.

*Weekly.* A single weekly dosage contains Vitamin D or 25-OH D3 in an amount from about 7 µg to about 350 µg, and preferably from about 35 to 175 µg. Alternatively, a single weekly dosage may contain both Vitamin D and 25-OH D3 each in an amount from about 7 µg to about 350 µg, and preferably from about 35 to 175 µg. The dosage ratio of Vitamin D to 25-OH D3 may be from about 50:1 to about 1:50, more preferably from about 25:1 to about 1:25, and even more preferably from about 6:1 to about 1:6.

*Monthly.* A single monthly dosage contains Vitamin D or 25-OH D3 in an amount from 30 µg to about 1500 µg, preferably about 75 µg to about 500 µg. Alternatively, a single monthly dosage may contain both Vitamin D and 25-OH D3 each in an amount from 30 µg to about 1500 µg, preferably about 75 µg to about 500 µg. A kit may be comprised of one, two, three, four, five, six, seven, eight, nine, ten, eleven, or twelve weekly or monthly dosages.

Dosage ratios of Vitamin D to 25-OH D3 should range between 50:1 to about 1:50, more preferably from about 25:1 to about 1:25, and even more preferably from about 6:1 to about 1:6.

### GENE ANALYSIS

To demonstrate increased bioactivity of the combination, a gene chip analysis of muscle tissue exposed to Vitamin D, 25-OH D3 and the combination was performed. Details are given in Example 2, using a murine hind-leg suspension model. As can be seen, there is a dramatic increase in the number of genes activated or regulated (either up-regulated or downregulated) when the combination of the two are delivered as compared to individual administration. As it is currently believed that the vast majority of Vitamin D is converted into 25-OH D upon ingestion and processing in the liver, this is a surprising result.

Described herein is a process of activating or regulating Vitamin D and 25-OH D responsive human muscle- related genes comprising administering to a person a combination of Vitamin D and 25-OH D3.

The following non-limiting Examples are presented to better illustrate the invention.

### EXAMPLES

### Example 1

### Clinical Trial

### Formulation

### Materials and Methods

Spray-dried formulation of 25-OH D3 was provided as a powder. In summary, 25-OH D3 and DL-α-tocopherol were dissolved in an oil of medium chain triglycerides, then emulsified into an aqueous solution of modified starch, sucrose, and sodium ascorbate. The emulsion was atomized in a spray dryer in the presence of silicon dioxide. The resulting powder was collected when water content (LDO) was less than 4% and sieved through 400µm. It was packed and sealed in alu-bags, then stored in a dry area below 15 °C and used within 12 months of its manufacture.

Three separate lots were manufactured. In detail, a matrix was produced by mixing for 120 min in a FRYMIX processing unit with an anchor stirrer at 70°C under vacuum and consisting of:
▪ 17.300 kg water (WBI)
▪ 13.460 kg modified food starch (CAPSUL HS)
▪ 3.270 kg sucrose
▪ 0.730 kg sodium ascorbate

An oil phase was prepared by mixing for 35 min in a double-walled vessel with propeller stirrer at 82°C and consisting of:
▪ 0.550 kg BERGABEST MCT oil 60/40
▪ 0.049 kg calcifediol (HY-D USP)
▪ 0.183 kg DL-α-tocopherol

The oil phase was transferred to the matrix in the FRYMIX processing unit and was pre-emulsified with its internal colloid mill (60 min, 70°C). The pre-emulsion was circulated through a high-pressure homogenizer (20 min). The emulsion with a viscosity of 60 mPa·s to 90 mPa· s at 70°C was transferred over the high pressure pump to the spray nozzle. As fluidizing agent, silicon dioxide (SIPERNAT 320 DS) was fed into the tower. The spraying and drying parameters are listed below.

| **Parameter** | **Spraying** | **Drying** |
|---|---|---|
| Inlet air position | top of tower | top of tower |
| Inlet air feed | 1500m³/h | 1400m³/h |
| Inlet air temperature | 170°C | heater switch off |
| IFB inlet air feed | 500m³/h | 500m³/h |
| IFB inlet air temperature | 65°C | 50°C |
| exhaust air position | bottom of the tower | bottom of the tower |
| fine powder recycling | to IFB | to IFB |
| emulsion feed rate | 50kg/h | emulsion feed stopped |
| SiO₂ feed position | top of tower | SiO₂ feed stopped |
| SiO₂ acid feed rate | 100g/h | SiO₂ feed stopped |

For each of the three lots of 25-OH D3, an average of 8.4 kg of spray-dried powder with about 0.25% content of 25-OH D3 was obtained. The other components of the formulation are: 73.2% modified food starch, 17.6% sucrose, 4.0% sodium ascorbate, 3.0% medium chain triglycerides, 1.0% silicon dioxide, and 1.0% DL-α-tocopherol.

Spray-dried formulation of vitamin D3 was provided as a powder. In summary, vitamin D3 and DL-α-tocopherol were dissolved in an oil of medium chain triglycerides, then emulsified into an aqueous solution of modified starch, sucrose, and sodium ascorbate. The emulsion was atomized in a spray dryer in the presence of silicon dioxide. The resulting powder was collected when water content (LOD) was less than 4% and sieved to remove big lumps. It was stored in a dry area below 15°C and used within 12 months of its manufacture.

### Clinical Trial

### Subjects

Healthy, postmenopausal women (50 to 70 years of age) were recruited using informed consent and screened using the following criteria: serum 25-OH D3 between 20 nmol/L and 50 nmol/L, body mass index between 18 kg/m² and 27 kg/m², blood pressure less than 146/95 mm Hg, serum calcium less than 2.6 nmol/L, fasting glucose less than 100 mg/dl, no high-intensity exercise more than three times per week, no treatment for hypertension, no use of high-dose vitamin D or calcium supplement or drug affecting bone metabolism (e.g., biphosphonate, calcitonin, estrogen receptor modulator, hormone replacement therapy, parathyroid hormone), and not visiting a "sunny" location during the study.

Subjects were randomly assigned to one of seven treatment groups (i.e., daily, weekly, bolus as single dose, and bolus as combination dose). Each group included five subjects. They are followed for four months in Zurich, Switzerland during the winter.

### Clinical Study

The objective was studying and comparing the pharmacokinetic characteristics of vitamin D3 and 25-OH D3 administered to humans. Equimolar quantities of both substances were investigated. The regimen is based on 20 µg/day (or its equivalent on a weekly basis) of 25-OH D3. As the maximum pre-existing baseline concentration of 25-OH D3 will be 50 nmol/L, it is not anticipated that subjects will approach the range where disturbance in Ca²⁺ homeostasis has been observed. For comparative purposes, it is necessary to administer equimolar quantities of either vitamin D3 or 25-OH D3. In respect to administration of vitamin D3, the dose is considered to be sufficient to overcome background variability and provide and efficacious dose to the participants.

**Daily: 120 administrations**

| | | |
|---|---|---|
| 1. | 25-OH D3 | 20 µg |
| 2. | Vitamin D3 | 20µg (800 IU) |

**Weekly: 16 administrations**

| | | |
|---|---|---|
| 3. | 25-OH D3 | 140µg |
| 4. | Vitamin D3 | 140µg (5600 IU) |

**Bolus: single administration**

| | | |
|---|---|---|
| 5. | 25-OH D3 | 140µg |
| 6. | Vitamin D3 | 140µg (5600 IU) |

**Bolus: combo administration**

| | | |
|---|---|---|
| 7. | D3 and 25(OH)D3 | 140µg (5600 IU) + 140µg |

Hard gel capsules, which are packaged in bottles, contain either 20 µg or 140 µg of either spray-dried vitamin D3 or 25-OH D3 per capsule. Each dosage is consumed orally at breakfast. The duration of the study is four months for the "Daily" and "Weekly" groups. Subjects enrolled in the "Bolus" group consume orally a single dosage at the second study visit.

Plasma concentrations of 25-OH D3 (e.g., peak and steady state) are determined by obtaining samples from the subjects at various times after the dosage is ingested. For screening purposes and to establish baseline values, a blood sample is obtained prior to enrollment into the study and the clinical laboratory measures vitamin D3, 25-OH D3, calcium, creatinine, albumin, and fasting glucose in the serum. On Monday of Week 1 of the study, pharmacokinetics of serum vitamin D3, 25-OH D3, and 1,25-dihydroxy vitamin D3; serum markers (i.e., vitamin D3, 25-OH D3, calcium, creatinine, albumin, PTH, GOT, GPT, ALP, triglycerides, HDL, LDL, total cholesterol, bALP, and fasting glucose); and urine markers (i.e., calcium, creatinine, and DPD) are assessed over 24 hours. Daily samples for the remaining days of Week 1 and Monday of Week 2 are taken to assess serum vitamin D3 and 25-OH D3, serum markers (i.e., calcium, creatinine, albumin), and urine markers (i.e., calcium, creatinine). The assessments continue on Monday of Weeks 3, 5, 7, 9, 11, 13 and 15. On Monday of Week 16, samples are taken to assess pharmacokinetics of serum vitamin D3, 25-OH D3, and 1,25-dihydroxy vitamin D3; serum markers (i.e., vitamin D3, 25-OH D3, calcium, creatinine, albumin, PTH, GOT, GPT, ALP, triglycerides, HDL, LDL, total cholesterol, bALP, and fasting glucose); and urine markers (i.e., calcium, creatinine, and DPD).

Muscle strength and function were assessed by the following standard performance tests: knee flexor and extensor strength, repeated sit-to-stand test and timed up & go (TUG) in Week 1 on visit 2 (baseline) and at study end on visit 15. Muscle strength was measured as knee extensor and flexor in Newtons (kiloponds). TUG is a measure of functional mobility including muscle strength, gait speed, and balance and is assessed in seconds. The repeated sit-to-stand is a functional test and measured in seconds.

### Results:

Table 1 shows the change in muscle strength after daily and weekly treatment with 25-OH D3 (20µg per day; 140µg per week, respectively) or daily and weekly treatment with Vitamin D3 (20µg per day; 140µg per week, respectively). Treatment duration was 4 months. Values are given as change after 4 months versus baseline (before start of treatment).

**TABLE 1**

| | **Muscle strength (Change vs. baseline in Newton)** | |
|---|---|---|
| | **Daily/weekly Vitamin D₃** | **Daily/weekly 25-OH D3** |
| Knee extension (mean value) | -23.7 | 13.0 |
| Knee extension (best value) | -24.8 | 13.6 |
| Knee flexion (mean value) | 3.6 | 7.9 |
| Knee flexion (best value) | 2.4 | 7.2 |

Table 2 shows the relative change in muscle strength after daily and weekly treatment with 25-OH D3 (20µg per day; 140µg per week, respectively) compared to daily and weekly treatment with Vitamin D3 (20µg per day; 140µg per week, respectively). Treatment duration was 4 months. Values are GLM (general linear model) least square means given as % improvement adjusted for baseline strength, age and body mass index for 25-OH D3 versus Vitamin D3.

**TABLE 2**

| | **Muscle strength (Change vs. Daily/weekly Vitamin D3 in %)** |
|---|---|
| | **Daily/weekly 25-OH D3** |
| Knee extension (mean value) | 17.0 |
| Knee extension (best value) | 17.6 |
| Knee flexion (mean value) | 3.1 |
| Knee flexion (best value) | 4.7 |

Table 3 shows the change in muscle function after daily and weekly treatment with 25-OH D3 (20µg per day; 140µg per week, respectively) or daily and weekly treatment with Vitamin D3 (20µg per day; 140µg per week, respectively). Treatment duration was 4 months. Values are given as time (in seconds) needed to complete the task after 4 months versus baseline (before start of treatment).

**TABLE 3**

| | **Muscle function (Change vs. baseline in Seconds)** | |
|---|---|---|
| | **Daily/weekly Vitamin D3** | **Daily/weekly 25-OH D3** |
| Repeated sit-to-stand | 0.30 | 0.63 |
| Timed-up-and-go | -0.46 | 0.27 |

Table 4 shows the relative change in muscle functions after daily and weekly treatment with 25-OH D3 (20µg per day; 140µg per week, respectively) compared to daily and weekly treatment with Vitamin D3 (20µg per day; 140µg per week, respectively). Treatment duration was 4 months. Values are GLM (general linear model) least square means given as % time needed to complete the task adjusted for baseline function, age and body mass index for 25-OH D3 versus Vitamin D3.

**TABLE 4**

| | **Muscle function (Change vs. Daily/weekly Vitamin D3 in %)** |
|---|---|
| | **Daily/weekly 25-OH D3** |
| Repeated sit-to-stand | 13.9 |
| Timed-up-and-go | 8.4 |

These data demonstrate that daily or weekly treatment with 25-OH D3 surprisingly results in much stronger improvements of muscle strength and function compared to consumption of identical dosages of Vitamin D3. After treatment with 25-OH D3, subjects were able to perform stronger knee extension and flexion compared to before treatment and compared to treatment with Vitamin D3. The relative improvement of muscle strength in subjects treated with 25-OH D3 versus Vitamin D3 was between 3 to 18%, an effect size that is clinically relevant and represents a significant benefit for subjects in all age groups and especially for postmenopausal females.

Muscle function determined by standard performance tests (repeated sit-to-stand, timed-up-and-go) was better in subjects treated with 25-OH D3compared to treatment with identical dosages of Vitamin D3. After treatment with 25-OH D3, subjects treated completed the performance tests faster compared to before treatment and compared to treatment with Vitamin D3. Relative improvements of muscle function after treatment with 25-OH D3 versus Vitamin D3 was between 8 to 14%, an effect size that is clinically relevant and represents a significant benefit for subjects in all age groups and especially for postmenopausal females.

Furthermore, an unadjusted analysis across the four lower extremity tests (knee extension, knee flexion, timed-up-and-go, and repeated-sit-to-stand) revealed that subjects treated with 25-OH D3 have a 2.8-fold higher likelihood of maintaining or improving lower extremity strength and function compared to subjects treated with identical dosages of Vitamin D3. This effect is statistically significant and clinically relevant and indicates that 25-OH D3 is suitable for treatments aimed at maintaining or improving skeletal muscle strength and function.

### EXAMPLE 2

### Gene chip data

The objective of this study was to test the effects of Vitamin D3, 25-OH D3, and the combination of Vitamin D3 and 25-OH D3 in a skeletal muscle atrophy model using BalbC mice where tail suspension leads to skeletal muscle atrophy in the unloaded hindlimbs of the animals. Initially this model was established in rats for simulating spaceflight in humans and is commonly used in other scientific fields to study the loss of skeletal muscle mass or bone. The results are considered indicative of human conditions such as sarcopenia (degenerative loss of skeletal muscle mass and strength during the process of ageing) or immobilization of skeletal muscle (e.g. after prolonged bed rest due to fractures, surgery or trauma).
*Methods* For our study, nine month old BalbC female mice were randomized at the beginning of the study into four groups with 10 animals per group
1. Control group: hindlimb unloaded (HU)
2. Vitamin D3 group: HU + treatment of Vitamin D3
3. 25-OH D3 group: HU + treatment of 25-OH D3
4. Vitamin D+ 25-OH D3 group: HU + treatment of Vitamin D3 and 25-OH D3 (combination)

The animals were placed in special cages for duration of seven days; all mice were housed separately and had free access to feed and water ad libidum. All animals were treated twice by gavage at the beginning of the experiment and 3 hours before the section:
1. the control group received vehicle (gelatine)
2. the D3 group received Vitamin D3 (50 µg/kg/bw),
3. the 25-OH D3 group received 25-OH D3 (50 µg/kg/bw)
4. the combination group received Vitamin D3 + 25-OH D3 (50 + 50 µg/kg/bw)

At the end of the study the gastrocnemius muscle was taken out and directly frozen in liquid nitrogen for further analysis. To identify changes in gene expression and analyse shifts in mRNA levels in the gastrocnemius muscle we used Affymetrix Mouse 430-2 microarrays together with the version 27 (December 2008) annotation files from Affymetrix for this array type. The array contains "45,000 probe sets to analyze the expression level of more than 39,000 transcripts and variants from more than 34,000 well-characterized mouse genes and UniGene clusters" (Affymetrix, 2009).

Total RNA was isolated using the commonly used Trizol protocol. The RNA was quantified by using spectrophotometric analysis. The integrity of total RNA samples was also assessed qualitatively on an Agilent 2100 Bioanalyzer. RNA was then prepared for the one cycle cDNA synthesis. A poly-A RNA control is used for this step to provide exogenous positive controls to monitor the entire eukaryotic target labelling process. The first cDNA synthesis is done, and after the second strand cDNA synthesis the cDNA is cleaned up of double-stranded cDNA. A biotin labelled cRNA is then synthesised, cleaned up and quantified using a spectrophotometer at 260/280 nm. It is important that cRNA target is fragmented before hybridization onto a GeneChip probe arrays to obtain optimal assay sensitivity. After fragmentation the probes are hybridized on the chips (Affymetrix Mouse 430-2 chips). The chips are washed and stained in the fluidics station of Affymetrix and scanned in the gene chip scanner. The data is then transferred from the scanner for further analysis using software from Genedata (Expressionist 5.0: Refiner Array and Analyst). Data interpretation and pathway analysis was done with the online version of the GeneGo Metacore package (V5.2 build 17389).

Refiner Array evaluates microarray data for quality issues and flags problematic measurements. It provides a set of normalization algorithms and validated condensing methods to automatically pre-process and summarize raw microarray data for subsequent statistical analysis.

Analysis of microarray data revealed genes (mRNAs) which were differentially expressed between HU group and HU plus treatment groups (Vitamin D3, 25-OH D3 or combination).

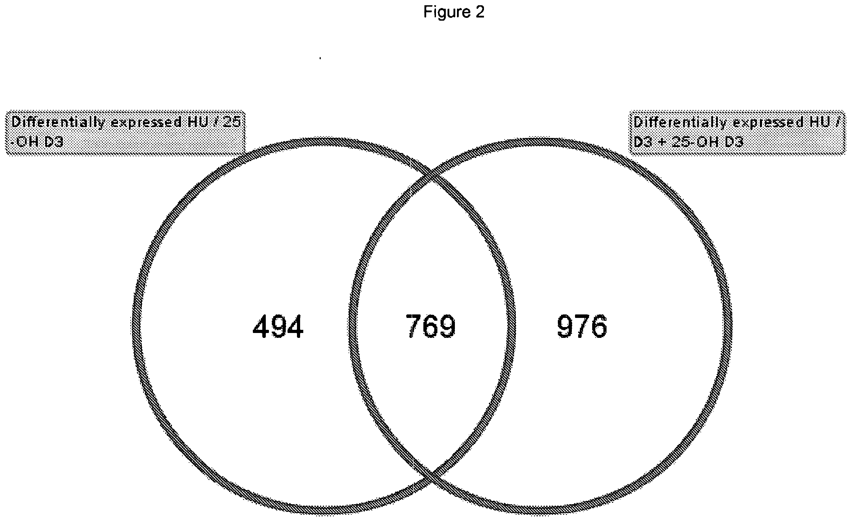

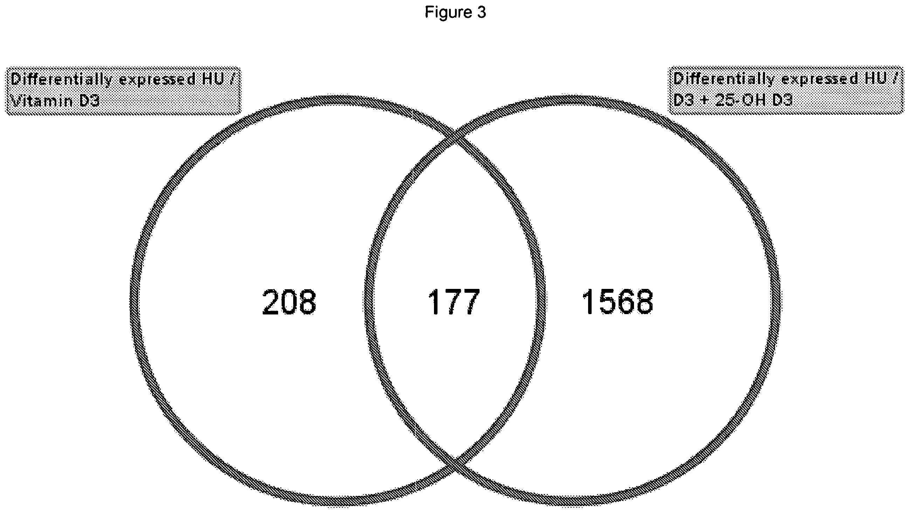

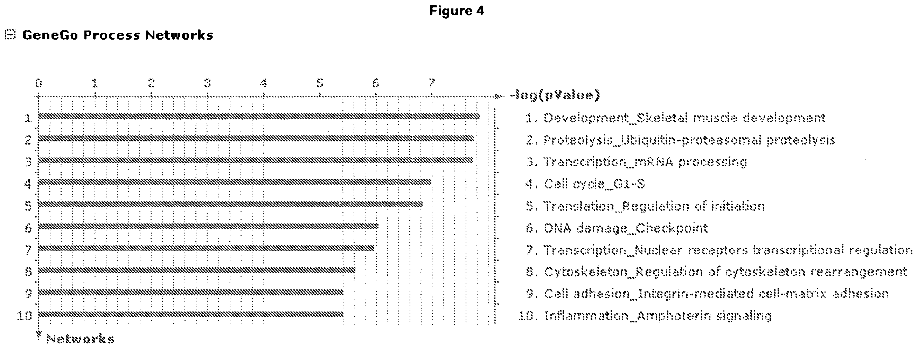

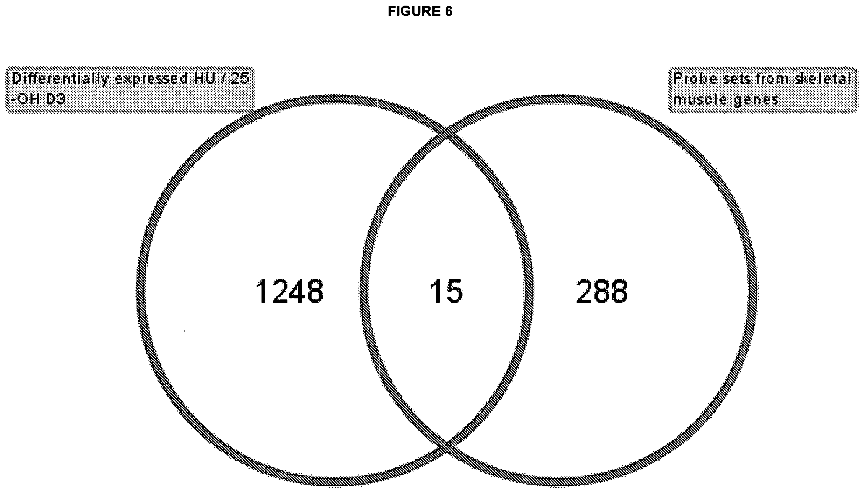

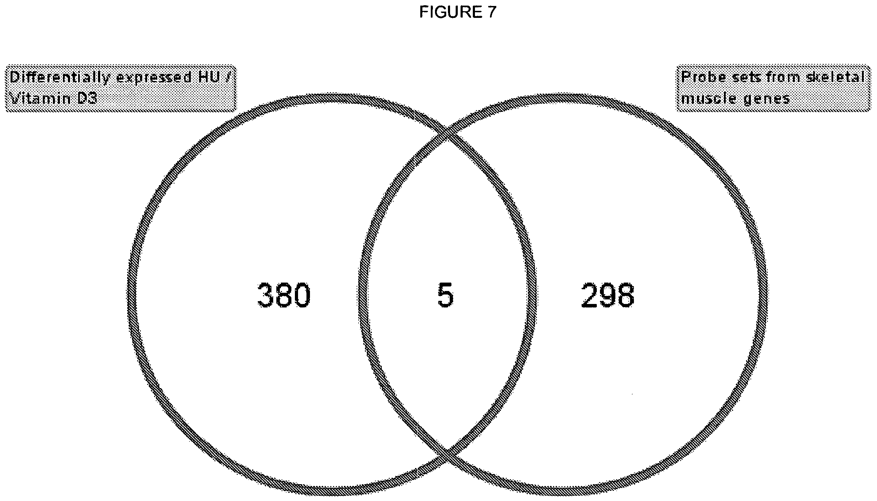

Our key findings are
1. A combination of 25-OH D3 and Vitamin D3 changes more probe sets for genes than 25-OH D3; which in turn changes more probe sets for genes than Vitamin D3 (Table ).
   a. compared to the HU control group, the group receiving a combination treatment (D3 + 25-Hydroxyvitamin D3) has significantly more probe sets for genes changed (1745) than the group which received a treatment with only 25-OH D3 (1263)
   b. compared to the HU control group, the group receiving a treatment with 25-OH D3 has significantly more probe sets for genes changed (1263) than the group which received a treatment with Vitamin D3 (385)Error! Reference source not found.)
2. A combination treatment of 25-OH D3 + Vitamin D3 has more common differentially expressed probe sets for genes with 25-OH D3 treatment than with Vitamin D3 treatment.
   a. ∼61% of the probe sets for genes differentially expressed in the 25-OH D3 group are also differentially expressed in 25-OH D3 + Vitamin D3 group (769 of 1263, Figure 2)
   b. ∼46% of the probe sets for genes differentially expressed in the Vitamin D3 group are also differentially expressed in the 25-OH D3 + Vitamin D3 group (177 of 385, Figure 3)
   c. A combination treatment of 25-OH D3 and Vitamin D3 has the most significant impact on the genes of the muscle development process, as illustrated in Figures 4, 6, and 7.
   d. Involved genes are part of the following main categories in skeletal muscle: muscle contraction, muscle development and muscle maintenance. (Table ).
3. For probe sets of selected genes of the skeletal muscle, a combination treatment of 25-OH D3 and Vitamin D3 shows higher expressions than treatments with only Vitamin D3 or only 25-OH D3 (Table)

**Table 5: Differentially expressed probe sets for genes between HU and the HU + treatment groups Vitamin D3, 25-OH D3, or combination.**

| **Parameter** | **Differentially expressed probe sets for genes** |
|---|---|
| Hindlimb unloading (HU - control) group | - |
| **Against** HU + Vitamin D3 | 385 |
| **Against** HU + 25-OH D3 | 1263 |
| **Against** HU + Vitamin D3 + 25-OH D3 | 1745 |

**Table 6: Differentially expressed probe sets for selected skeletal muscle genes between HU and the treatment groups**

| **Parameter** | **Differentially expressed muscle genes compared to HU** |
|---|---|
| HU + Vitamin D3 | Desmin; myoneurin; tropomyosin 1, alpha; tropomyosin 2, beta; X-linked myotubular myopathy gene 1 |
| HU + 25-OH D3 | Desmin; dystonin; myocyte enhancer factor 2A; myoneurin; myosin VIIb; myosin, light polypeptide 6, alkali, smooth muscle and non-muscle; myosin, light polypeptide kinase; myotubularin related protein 2; myotubularin related protein 4; myotubularin related protein 6; tropomyosin 2, beta; X-linked myotubular myopathy gene 1 |
| HU + Vitamin D3 + 25-OH D3 | calsequestrin 2; desmin; dystonin; dystrophin, muscular dystrophy; myocyte enhancer factor 2A; myocyte enhancer factor 2C; myocyte enhancer factor 2D; myomesin 1; myoneurin; myosin X; myosin, heavy polypeptide 6; myosin, heavy polypeptide 8; myosin, light polypeptide 6; myotubularin related protein 1; myotubularin related protein 2; myotubularin related protein 4; myotubularin related protein 6; nebulin; sarcoglycan, beta; similar to myosin, heavy polypeptide 4; titin; tropomyosin 2, beta; troponin C; troponin I; X-linked myotubular myopathy gene 1 |

**5 Table 7: Expression pattern for selected genes of the skeletal muscle**

| | HU | Vitamin D3 | 25-OH D3 | 25-OH D3 + Vitamin D3 |
|---|---|---|---|---|
| Tropomyosin 1 (Tpm1) | 1179 | 2199 | 2110 | 2456 |
| Myosin, light chain kinase (Mylk) | 749 | 786 | 994 | 1022 |
| Myomesin 1 (Myom 1) | 84697 | 99099 | 105393 | 114398 |
| Titin (Tnt) | 11353 | 11354 | 13786 | 15647 |

### DISCUSSION REGARDING SPECIFIC SELECTED GENES

During one's life span, the skeletal muscles are permanently adapting to different stimuli, such as physical exercise and training, but also to immobilization. The skeletal muscle responds with either hypertrophy or atrophy. The development and adaptation of the skeletal muscle is a complex process. Briefly, satellite cells - the so called stem cells of the skeletal muscle - receive stimuli and form undifferentiated myoblasts which undergo fusion to form myotubes - new muscle fibers.

For the movements and the adaptation of the skeletal muscle, contraction is important. Skeletal muscle contraction is a mutual sliding of the two main skeletal muscle fibers myosin (thick filaments) and actin (thin filaments) which are organized in sarcomeres. They give skeletal muscles its cross striated appearance in the microscope.

Beside the thin and the thick filaments the skeletal muscle is composed of titin and nebulin and also sarcomeric proteins such as tropomyosin. Skeletal muscle function depends on a precise alignment of the actin and myosin filaments and the accessory proteins such as α-actinin, myomesin, M-protein, titin, desmin and myosin binding proteins.

It has been suggested that myomesin and M-protein may connect titin and myosin filament systems and that myomesin plays a role in integrating thick filaments into assembling sarcomeres. Titin, which is a huge protein, forms a continuous filament system in myofibrils. The predominant intermediate filament protein of striated muscle is desmin, and contributes to maintaining the integrity and alignment of myofibrils.

Mutations in several protein components (e.g. myosin heavy chain, actin, tropomyosin etc.) and also sarcomer proteins (e.g. titin, desmin etc.) are associated with muscle diseases/myopathies.

### Tropomyosin 1, alpha (Tpm1):

As stated on WIKIPEDIA,
"Tropomyosin is an actin-binding protein that regulates actin mechanics. It is important, among other things, for muscle contraction. Tropomyosin, along with the troponin complex, associates with actin in muscle fibers and regulate muscle contraction by regulating the binding of myosin. In resting muscle, tropomyosin overlays the myosin binding sites on actin and is "locked" down in this position by troponin T (tropomyosin binding troponin) and troponin I (inhibitory troponin). Upon release of calcium from the sarcoplasmic reticulum calcium binds to troponin C (calcium binding troponin). This "unlocks" tropomyosin from actin, allowing it to move away from the binding groove. Myosin heads can now access the binding sites on actin. Once one myosin head binds, this fully displaces tropomyosin and allows additional myosin heads to bind, initiating muscle shortening and contraction. Once calcium is pumped out of the cytoplasm and calcium levels return to normal, tropomyosin again binds to actin, preventing myosin from binding."

Tropomyosin 1 alpha is a gene which is required for development and muscle function (e.g. muscle contraction). In general the muscle-specific Tropomyosins regulate actin-myosin interactions and hence contraction. The encoded protein is one type of alpha helical chain that forms the predominant tropomyosin of striated muscle, where it also functions in association with the troponin complex to regulate the calcium-dependent interaction of actin and myosin during muscle contraction. (NCBI)

### Expression pattern (Tpm1):

| HU | HU+VitD3 | HU+25-Hydroxyvitamin D3 | HU+VitD3+25-Hydroxyvitamin D3 |
|---|---|---|---|
| 1179 | 2199 | 2110 | 2456 |

### Example from the 25-Hydroxyvitamin D3 treated group:

### Myosin, light chain kinase (MYLK):

Wikipedia:
"Mylk is a human gene. This gene, a muscle member of the immunoglobulin gene superfamily, encodes myosin light chain kinase which is a calcium/calmodulin dependent enzyme. This kinase phosphorylates myosin regulatory light chains to facilitate myosin interaction with actin filaments to produce contractile activity. This gene encodes both smooth muscle and nonmuscle isoforms. In addition, using a separate promoter in an intron in the 3' region, it encodes telokin, a small protein identical in sequence to the C-terminus of myosin light chain kinase that is independently expressed in smooth muscle and functions to stabilize unphosphorylated myosin filaments. A pseudogene is located on the p arm of chromosome 3. Four transcript variants that produce four isoforms of the calcium/calmodulin dependent enzyme have been identified as well as two transcripts that produce two isoforms of telokin. Additional variants have been identified but lack full length transcripts."

### Expression pattern (MYLK):

| HU | HU+VitD3 | HU+25-Hydroxyvitamin D3 | HU+VitD3+25-Hydroxyvitamin D3 |
|---|---|---|---|
| 749 | 786 | 994 | 1022 |

### Example from the Vitamin D3 + 25-Hydroxyvitamin D3 treated group:

Wikipedia:
Myomesin is an end line protein that is part of the M line. It attaches myosin thick filaments at the M line.
Expression pattern (Myomesin 1 - Myom1):

| HU | HU+VitD3 | HU+25-Hydroxyvitamin D3 | HU+VitD3+25-Hydroxyvitamin D3 |
|---|---|---|---|
| 84697 | 99099 | 105393 | 114398 |

### Wikipedia:

"Titin, also known as connectin is a protein that is important in the contraction of striated muscle tissues. Titin is a large abundant protein of striated muscle. The protein is divided into two regions:
N-terminal I-band - is the elastic part of the molecule, contains two regions of tandem immunoglobulin domains on either side of a PEVK region that is rich in proline, glutamate, valine and lysine
C-terminal A-band - thought to act as a protein-ruler, contains a mixture of immunoglobulin and fibronectin repeats, and possesses kinase activity. A N-terminal Z-disc region and a C-terminal M-line region bind to the Z-line and M-line of the sarcomere respectively so that a single titin molecule spans half the length of a sarcomere. Titin also contains binding sites for muscle associated proteins so it serves as an adhesion template for the assembly of contractile machinery in muscle cells. It has also been identified as a structural protein for chromosomes. Considerable variability exists in the I-band, the M-line and the Z-disc regions of titin. Variability in the I-band region contributes to the differences in elasticity of different titin isoforms and, therefore, to the differences in elasticity of different muscle types. Of the many titin variants identified, five for which complete transcript information is available are described."

Titin interacts with many sarcomeric proteins including:
Z line region: telethonin and alpha-actinin
I band region: calpain-3 and obscurin
M line region: myosin-binding protein C, calmodulin 1, CAPN3, and MURF1

### Expression pattern (Titin - Tnt):

| HU | HU+VitD3 | HU+25-Hydroxyvitamin D3 | HU+VitD3+25-Hydroxyvitamin D3 |
|---|---|---|---|
| 11353 | 11354 | 13786 | 15647 |

### Example 3

### Mouse Model

The objective of this study was to test the effects of 25-OH D3 in a muscle hypertrophy model and in an endurance exercise capacity test in C57BL/6J mice. It is recognized in the art that removal of the gastrocnemius muscle induces compensatory hypertrophy in the soleus and plantaris muscles by multiple mechanisms leading to improved muscle strength and leg power.

Two groups of 10 animals were anesthetized and the left hindlimb of the animals was fixed. All animals received an analgesic. A small incision was made through the skin over the gastrocnemius muscle. The complete gastrocnemius muscle and the tendons were exposed. Both heads of the gastrocnemius muscle were carefully dissected from the underlying intact muscles and care was taken not to rupture nerves and vessels. The skin was closed with a silk suture and the animals were returned into the cages. After recovering from anesthesia the animals could move directly without problems in their cages. Animals were treated for three weeks by gavage with 25-OH D3 at a daily dosage of 50 µg/kg and the control group received vehicle. At the end of the study endurance exercise capacity of all animals was tested on a rodent treadmill.

The wet weight of the soleus and plantaris muscles were increased in animals treated with 25-OH D3 compared to control animals (Table 8). Furthermore, when muscle weights were normalized to the body weights of in mice compared to the body weight, animals treated with 25-OH D3 demonstrated an increased soleus-plantaris muscle weight / body weight ratio (Table 8). Computer tomography measurements of muscle and total leg area confirmed that 25-OH D3 treatment increases skeletal mass (Table 8). Animals receiving 25-OH D3 displayed increased endurance exercise capacity compared to control mice demonstrated by longer running distance and time (Table 8).

Table 8 shows muscle weights, muscle weight / body weight ratios, total leg and muscle cross-sectional areas, running distance and running time of mice treated with 25-OH D3 at a dosage of 50 µg/kg/day or placebo (control) for 3 weeks.

**TABLE 8**

| **Parameters** | **Control** (n=10) | **25-OH D3** (n=10) |
|---|---|---|
| Soleus wet weight (mg) | 5.25 | 6.37 |
| Plantaris wet weight (mg) | 31.68 | 33.24 |
| Soleus-Plantaris muscle weight / body weight (mg/g) | 1.82 | 1.94 |
| Total leg cross-sectional area (mm²) | 22.78 | 24.04 |
| Muscle cross-sectional area (mm²) | 19.90 | 20.98 |
| Running distance (m) | 986.0 | 1078.4 |
| Running time (min) | 37.6 | 41.0 |

## Claims

1. Use of 25 hydroxyvitamin D3 ("25-OH D3") and Vitamin D3 in the manufacture of a pharmaceutical, nutraceutical, food supplement or food composition to retain or prevent the loss of muscle function or muscle strength in a human, where the human is an elderly person, or a person who suffers chronic immobility regardless of age.

2. Use according to claim 1 wherein the 25-OH D3 and Vitamin D3 are present in separate dosage units.

3. Use according to Claim 1 or 2 wherein 25-OH D3 and Vitamin D3 are used once per day, once per week, or once per month.

4. Use according to any of Claims 1-3 wherein the usage is for at least one month, preferably for more than two months, and more preferably for at least four months.

5. Use according to any of claims 1-4 wherein the ratio of Vitamin D3 to 25-OH D3 is from 6:1 to 1:6.

## Patentansprüche

1. Verwendung von 25-Hydroxyvitamin D3 ("25-OH-D3") und Vitamin D3 bei der Herstellung eines Arzneimittels, Nutraceuticals, Nahrungsergänzungsmittels oder einer Lebensmittelzusammensetzung zum Erhalten der Muskelfunktion oder Muskelstärke oder Vorbeugen des Verlusts davon beim Menschen, wobei es sich bei dem Menschen um eine ältere Person, oder eine Person, die unabhängig vom Alter an chronischer Immobilität leidet.

2. Verwendung nach Anspruch 1, wobei das 25-OH-D3 und Vitamin D3 in getrennten Dosierungseinheiten vorliegen.

3. Verwendung nach Anspruch 1 oder 2, wobei das 25-OH-D3 und Vitamin D3 einmal pro Tag, einmal pro Woche oder einmal pro Monat angewandt werden.

4. Verwendung nach einem der Ansprüche 1-3, wobei die Anwendung wenigstens einen Monat, vorzugsweise mehr als zwei Monate und stärker bevorzugt wenigstens vier Monate dauert.

5. Verwendung nach einem der Ansprüche 1-4, wobei das Verhältnis von Vitamin D3 zu 25-OH D3 6:1 bis 1:6 beträgt.

## Revendications

1. Utilisation de 25-hydroxy-vitamine D3 ("25-OH-D3") et de vitamine D3 dans la préparation d'une substance pharmaceutique, d'une substance nutraceutique, d'un complément alimentaire ou d'une composition alimentaire afin de conserver ou empêcher la perte de fonction musculaire ou de force musculaire chez un être humain, où l'être humain est une personne âgée, ou une personne qui souffre d'immobilité chronique indépendamment de son âge.

2. Utilisation selon la revendication 1, dans laquelle la 25-OH D3 et la vitamine D3 sont présentes dans des unités de dosage séparées.

3. Utilisation selon la revendication 1 ou 2, dans laquelle la 25-OH D3 et la vitamine D3 sont utilisées une fois par jour, une fois par semaine, ou une fois par mois.

4. Utilisation selon l'une quelconque des revendications 1-3, dans laquelle l'usage est prévu pour au moins, préférablement pur au moins quatre mois.

5. Utilisation selon l'une quelconque des revendications 1-4, dans laquelle le rapport de la vitamine D3 à la 25-OH D3 va de 6:1 à 1:6.
